# EUROPEAN PATENT APPLICATION

(11) **EP 1 865 432 A2**
(43) Date of publication of application: **12.12.2007**
(21) Application number: 07011271.9
(22) Date of filing: 08.06.2007
(51) Int. Cl.: G06F 19/00

(54) **Medical information management apparatus for managing interview sheet data**

(30) Priority: 09.06.2006 JP 2006161608
(71) Applicant: OLYMPUS MEDICAL SYSTEMS CORP., Shibuya-ku Tokyo 151-0072 (JP)
(72) Inventor: Tsuruma, Hidenori, Hachioji-shi Tokyo 192-0906 (JP); Matsubara, Nobuaki, Saitama-shi Saitama 338-0001 (JP)
(74) Representative: von Hellfeld, Axel

(57) **Abstract**

A recording unit (30) records implementation information on a medical treatment performed on a patient. An extraction unit (22) extracts information necessary to prepare an interview result of an interview item for the patient, from the implementation information recorded in the recording unit (30). A preparation unit (24) prepares the interview result by referring to the information extracted by the extraction unit (22), and prepares interview sheet data from the interview result and the interview item corresponding to the interview result.

## Description

The present invention relates to a medical information management technology and, more particularly, to a medical information management apparatus for managing interview sheet data.

At a medical institution, a patient is typically interviewed before any medical treatment is given to him/her. In such an interview, a physician asks the patient about his/her complaint or condition of a disease, past medical history, family medical history, and the like to gain some clues for diagnosis. In many cases, a typical practice is such that an interview sheet is given to the patient and the patient puts down his/her answers to the questions on the interview sheet. Then the physician checks the interview sheet recovered from the patient before the physician performs a medical action on him/her, and the interview sheet is either stored together with the patient's chart or inputted to a computer and recorded as electronic data. When the interview sheet is recorded as electronic data, however, it is necessary to input the interview information on each new patient right from the beginning, which imposes a heavy burden on medical personnel.

Reference (1) in the following Related Art List discloses an electronic patient's chart system to enhance the efficiency of medical treatment by physicians by automatically introducing necessary information into a patient's chart from interview information which is inputted beforehand through an interview system in coordination with the existing interview system.

### Related Art List

(1) Japanese Patent Application Laid-Open No. Hei10-323329.

As with the case of Reference (1), it is possible to prepare an interview system and ask a patient to input interview information. However, introducing a new interview system involves considerable cost. Also, the necessity for patients to learn the operation of the system may result in the production of interview sheet data which may include their erroneous answers due to mistaken input or faulty memory.

The present invention has been made in view of the foregoing circumstances, and a general purpose thereof is to provide a medical information management apparatus capable of preparing reliable interview sheet data while reducing the trouble of medical personnel in the recording of interview results.

In order to resolve the above problems, a medical information management apparatus according to one embodiment of the present invention comprises: a recording unit which records implementation information on a medical action performed on a patient; an extraction unit which extracts information necessary to prepare an interview result of an interview item for the patient, from the implementation information recorded in the recording unit; and a preparation unit which prepares the interview result by referring to the information extracted by the extraction unit. The preparation unit may prepare interview sheet data from the interview result and the interview item corresponding thereto.

According to this embodiment, the interview result is prepared by using the implementation information such as examination data. Thus, while reducing the trouble of medical personnel in the recording of interview results, highly reliable interview sheet data can be prepared.

The recording unit may record notandum information for the patient, and the extraction unit may extract information necessary for preparing the interview result, by using the notandum information. The "notandum information" or "notes information" may serve to provide a medical action performer with knowledge about forbidden medication, medical treatment and such other conditions for a patient and may be information to draw the medical personnel's attention thereto. If the notandum information for a patient is recorded in the recording unit, the interview result for interview items related thereto can be prepared by using the notandum information. Thus the interview results for the further increased number of interview items can be prepared.

Another embodiment of the present invention relates also to a medical information management apparatus. This apparatus comprises: a table which manages a medical action and an interview result for an interview item derived from the medical action by associating the medical action with the interview result; a search unit which conducts a search on whether a medical action performed on a patient corresponds to the medical action managed by the table or not; and a preparation unit which prepares interview sheet data from the interview result associated with the medical action and the interview item corresponding thereto when there exists the corresponding medical action as a result of the search by the search unit.

According to this embodiment, the interview result can be easily obtained from implementation information by using the table which manages the interview result for an interview item by associating it with the medical action.

The apparatus may further comprising a patient-related information generator which generates patient-related information for each patient by extracting notandum information for a patient contained in at least one of the interview sheet data, the implementation information, report information and order information. Thereby, the medical action performer can easily utilize the notandum information by managing the notandum information in a unified manner.

When conflicting notandum information is extracted from the interview sheet data, the implementation information, the report information and the order information, the patient-related information generator may give priority to safer information for the patient among the conflicting notandum information. As a result, when a medical action performer performs a medical action by using the notandums, he/she can protect the safety of patients more.

The apparatus may further comprise a relatives information acquiring unit which acquires medical information concerning patient's relatives; and a family medical history information generator which generates family medical history information based on the acquired medical information concerning patient's relatives. The preparation unit may prepare the interview sheet data by referring to the interview result, the interview item corresponding thereto and the family medical history information. As a result, by utilizing family medical history information, an interview result for the interview items related to the family medical history information can be prepared. Thus the interview results for the further increased number of interview items can be prepared.

It is to be noted that any arbitrary combination of the above-described structural components or rearrangement in the form among a method, an apparatus, a system, a recording medium, a computer program and so forth are all effective as and encompassed by the present embodiments.

Embodiments will now be described by way of examples only, with reference to the accompanying drawings which are meant to be exemplary, not limiting.
FIG. 1 illustrates a structure of a medical information management system according to a first embodiment of the present invention;
FIG. 2 illustrates an example of interview sheet;
FIG. 3 shows an example of a table associating medical actions with consequences derived from the medical actions;
FIG. 4 shows an example of interview sheet data;
FIG. 5 is a flowchart to explain an operation of a medical information management apparatus according to the first embodiment of the present invention;
FIG. 6 illustrates a structure of a medical information management apparatus according to a second embodiment of the present invention;
FIG. 7 shows an example of a patient information database which is a database of patient information;
FIG. 8 shows an example of an order information database which is a database of order information;
FIG. 9 shows an example of an implementation information database which is a database of implementation information;
FIG. 10 shows an example of an interview sheet database, which is a database of interview sheet data;
FIG. 11 shows an example of a report information database which is a database of report information;
FIG. 12 shows an example of a patient-related information database which is a database of patient-related information;
FIG. 13 shows a priority order table;
FIG. 14 shows an example of a relatives information database which is a database of relatives information; and
FIG. 15 is a flowchart to explain an operation of a medical information management apparatus according to the second embodiment of the present invention.

The invention will now be described by reference to the preferred embodiments. This does not intend to limit the scope of the present invention, but to exemplify the invention.

An outline of the present invention will be given before a specific description thereof. A medical information management apparatus according to a preferred embodiment of the present invention manages interview sheet data. The medical information management apparatus prepares interview sheet data by utilizing information on medical actions performed. As a result, a database can be created more easily than when a nurse or the like manually inputs patient's answers to interview items written on paper. Moreover, the database can be based on highly reliable information reflecting the results of medical action by a physician rather than the input from a terminal by a patient.

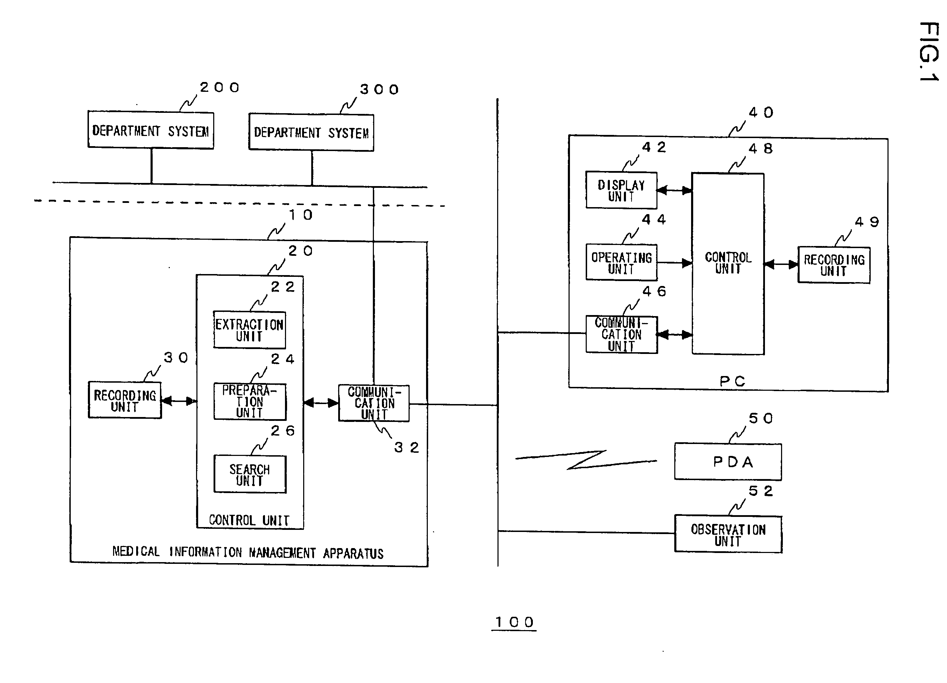

Hereinbelow, embodiments of the present invention will be described in detail. FIG. 1 illustrates a structure of a medical information management system 100 according to a first embodiment of the present invention. The medical information management system 100 includes a medical information management apparatus 10, a PC 40, a PDA (Personal Digital Assistant) 50, and an observation unit 52. These components, which are connected to a network spread out from a hub, constitute a LAN (Local Area Network). The PDA 50 is connected to the LAN wirelessly via a not-shown access point. The observation unit 52 corresponds to an electronic endoscope unit, an ultrasonic endoscopic observation unit, an extracorporeal echo unit, an external camera unit, or the like. The PC 40 and the PDA 50 function as client equipment for the medical information management apparatus 10. The following description will be given by taking, as an example, a management system in an endoscopic department which serves as a medical information management system 100.

The medical information management apparatus 10 includes a control unit 20, a recording unit 30, and a communication unit 32. The control unit 20 includes an extraction unit 22, a preparation unit 24, and a search unit 26. The PC 40 includes a display unit 42, an operating unit 44, a communication unit 46, a control unit 48, and a recording unit 49.

In terms of hardware, this structure described as above can be realized by a CPU, a memory and other LSIs of an arbitrary computer. In terms of software, it can be realized by memory-loaded programs or the like, but drawn and described herein are function blocks that are realized in coordination with those. Hence, it is understood by those skilled in the art that these function blocks can be realized in a variety of forms such as by hardware only, software only or the combination thereof.

The medical information management apparatus 10, which controls an endoscopic department system in a unified manner, manages not only patient information and implementation information but also interview sheet data. The recording unit 30, which comprises a storage medium such as a hard disk, manages patient information, order information, implementation information, report information, interview sheet data, and tables associating medical actions with the consequences derived from the medical actions, as its constituent databases. The databases and tables will be discussed in detail later. The communication unit 32 performs communication with the PC 40, the PDA 50, and the observation unit 52. The communication unit 32 also performs communication with other department systems 200 and 300.

The control unit 20 controls the medical information management apparatus 10 as a whole in a unified manner. According to the present embodiment, the control unit 20 prepares interview sheet data from implementation information by referring mainly to the tables associating medical actions with the consequences derived from the medical actions. The extraction unit 22 extracts a medical action performed on a patient from the implementation information. The search unit 26 makes a search to find if the extracted medical action corresponds to any of the medical actions managed by the table associating medical actions with the consequences derived from the medical actions. If any corresponding medical action is found in the above-mentioned table, the preparation unit 24 will determine the consequence associated therewith to be the result of interview, prepare interview sheet data using said result of interview and the interview item corresponding thereto, and record the prepared data in the recording unit 30.

The PC 40 can access the medical information management apparatus 10 and thereby reference or process patient information, order information, implementation information and so forth. The display unit 42 displays patient information, implementation information or the like. The operating unit 44, which is provided with a keyboard or the like, accepts input from the user. The communication unit 46 performs communication with the medical information management apparatus 10, the PDA 50, and the observation unit 52. The control unit 48 controls the PC 40 as a whole in a unified manner. The recording unit 49, which is comprised of a storage medium such as a hard disk, records report information and the like.

The PDA 50, which is to be carried by each of nurses and other medical personnel, has basically the same structure as the PC 40. It is further provided with a reading unit, which is comprised of a not-shown handy scanner or the like capable of reading identification information on medication, equipment, patients' wristbands and so forth. The observation unit 52 is a device which is used in performing an examination or treatment on an organ or the inside of a coelom of a patient. The observation unit 52 transmits picked-up images or the like as examination data to the medical information management apparatus 10.

The implementation information as mentioned above is information concerning the performance of examination or treatment, which may include "examination ID" or "treatment ID", "examination date" or "treatment date", "performer", "place of performance", "procedure", "medication", "equipment", "picked-up images", "schema images (schematic images)" and the like as appropriate.

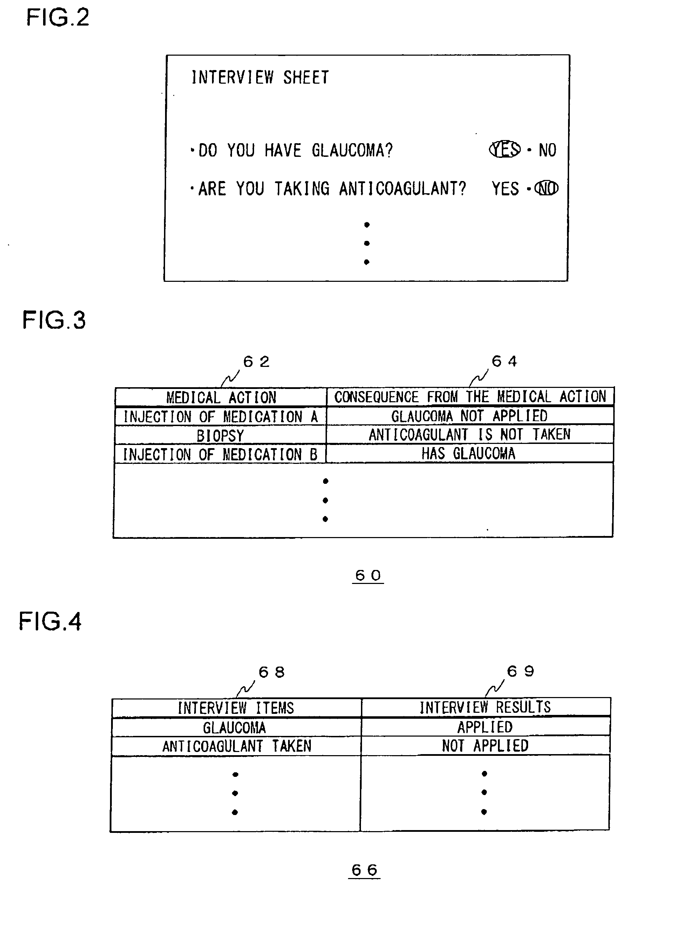

FIG. 2 illustrates an example of interview sheet. An interview sheet is a document listing question items for a patient and his/her answers thereto. The case of FIG. 2 is an example of interview to be performed prior to an endoscopic examination, and it is asking the patient whether he/she has glaucoma or not and whether he/she is taking any anticoagulant or not. The physician carries out an examination or treatment by consulting an interview sheet like this. For example, if the patient has glaucoma, the physician can see that there is some medication the physician must avoid. If the patient is taking some anticoagulant, the physician may not decide on an immediate excision of a polyp in the digestive tract, even if such a polyp is discovered during the endocopic examination, because the bleeding may not stop from such an excision.

FIG. 3 shows an example of a table 60 associating medical actions with consequences derived from the medical actions. In the example of FIG. 3, when there is an injection of medication A as a medical action 62, one can see that the patient does not have glaucoma as a consequence 64 derived from such medical action. Also, from the performance of a biopsy, one can deduce that the patient is not taking any anticoagulant. Likewise, from the injection of medication B, one can see that the patient has glaucoma.

FIG. 4 shows an example of interview sheet data. The interview sheet data 66 includes interview items 68 and interview results 69. From the second time of medical examination on, a medical action performer can consult the interview sheet data prepared at the first examination, using a PC 40 or a PDA 50. Also, the medical action performer can produce a paper interview sheet by printing interview sheet data out from the PC 40.

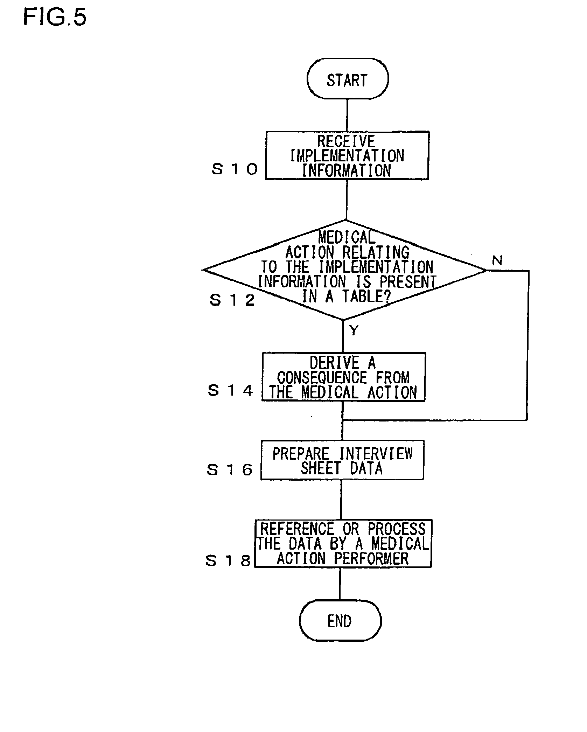

FIG. 5 is a flowchart to explain an operation of a medical information management apparatus 10 according to the first embodiment. Firstly, the medical information management apparatus 10 receives implementation information from the observation unit 52 (S10). The search unit 26 makes a search to find if a medical action contained in the received implementation information is present in the table 60 associating medical actions with the consequences derived from the medical actions (S12). If it is present (Y of S12), the preparation unit 24 extracts an applicable consequence derived from the medical action from the table 60 (S14) and prepares interview sheet data for the interview items corresponding to said consequence (S16). If it is not present (N of S12), the preparation unit 24 prepares interview sheet data in such a manner that the answers to the interview items are blank (S16). The processing in steps S12 to S16 is that for a single medical action contained in implementation information. Where a plurality of medical actions are contained therein, the processing in steps S12 to S16 is performed for each of the medical actions.

Each of medical action performers can reference or process the interview sheet data by using the PC 40 or the PDA 50 (S18). For example, if there is a paper interview sheet with an answer to an interview item which was not available from implementation information, he/she can enter the data manually. Also, when there is any change in the interview items or interview results, he/she can enter the correction manually.

According to the present embodiment as described above, the medical information management apparatus prepares interview sheet data based on the implementation information on medical actions. Therefore it is not necessary to manually input the contents of a paper interview sheet which medical action performers have asked the patient to fill out, thus saving the time for preparing interview sheet data. Also, the interview sheet data thus prepared reflects the diagnosis of a physician, so that the accuracy of the contents can be improved. For example, there are cases where aged patients may have forgotten or have a mistaken idea about the medication to be avoided or their past medical history. In this regard, the present embodiment, in which the physician prepares interview sheet data based on the information from diagnoses, provides better accuracy than the patient's self assessment.

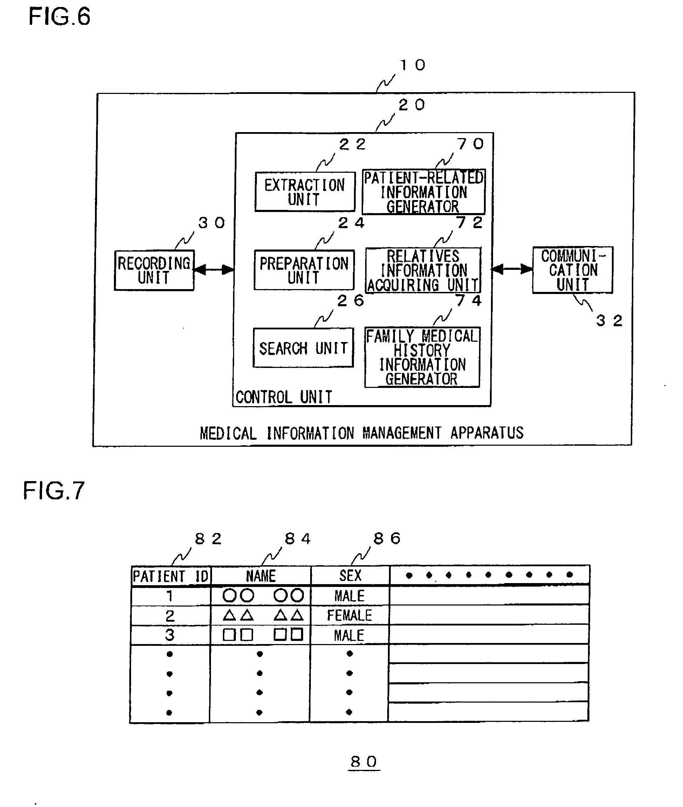

FIG. 6 illustrates a structure of a medical information management apparatus 10 according to a second embodiment of the present invention. The structure of the medical information management apparatus 10 according to the second embodiment is basically the same as that of he medical information management apparatus 10 according to the first embodiment. Therefore, only differences thereof will be explained hereinbelow. The control unit 20 according to the second embodiment includes an extraction unit 22, a preparation unit 24, a search unit 26, a patient-related information generator 70, a relatives information acquiring unit 72, and a family medical history information generator 74. The patient-related information generator 70 generates patient-related information to be described later. The relatives information acquiring unit 72 acquires relatives information concerning the patient's relatives from a relatives information database. The family medical history information generator 74 generates the patient's family medical history information based on the acquired relatives information. These operations will be discussed in detail later.

A description will now be given of a technique for generating patient-related information using various databases recorded in the recording unit 30. The interview sheet database to be described hereinbelow may be either one prepared according to the first embodiment or one prepared by some other technique.

The recording unit 30 manages a patient information database, an order information database, an implementation information database, an interview sheet database, a report information database, a patient-related information database, a priority order table, relatives information database and so forth.

FIG. 7 shows an example of a patient information database 80, which is a database of patient information. The patient information database 80 manages the names 84 and sexes 86 of the patients, using patient IDs 82 as the key. Patient information, which is information for identifying each individual patient, includes such items as "patient ID", "name", "sex", and "birth date". The "patient ID" is a number corresponding uniquely to a patient so as to identify the patient in distinction from the others. Further as patient profile information showing the characteristics and conditions of a patient, the patient ID may include such items as "blood type", "height/weight", "allergy", "disorder", "infection", "disease & notandum", "laboratory test results", "previous medication information" and so forth.

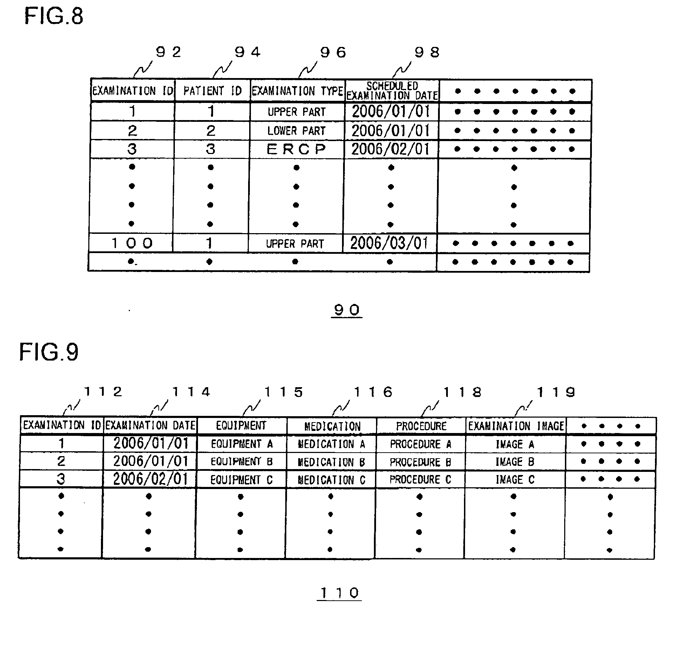

FIG. 8 shows an example of an order information database 90, which is a database of order information. The order information database 90 manages patient IDs 94, examination types 96, scheduled examination dates 98, and the like for each examination, using examination IDs 92 as the key. The order information is information concerning orders and requests for examinations to be conducted to a hospital department, such as the endoscopic department. The order information includes order key information, such as "examination ID", "patient ID", "name", "order No.", and "order date", requester information, such as "requesting department name", "requesting physician name", and "requesting date", order contents, such as "requested disease name", "examination purpose", "examination type", "examination items", "examination regions", and "comment", and examination booking information, such as "scheduled examination date" and "implementation time". The order key information is information for identifying each examination order in distinction from the others.

FIG. 9 shows an example of an implementation information database 110, which is a database of implementation information. The implementation information database 110 manages examination dates 114, equipment 115, medications 116, procedures 118, examination images 119, and the like for each examination, using examination IDs 112 as the key. Note that although a database for managing examinations is shown in FIG. 9, a database for managing treatments may also be used to manage implementation information.

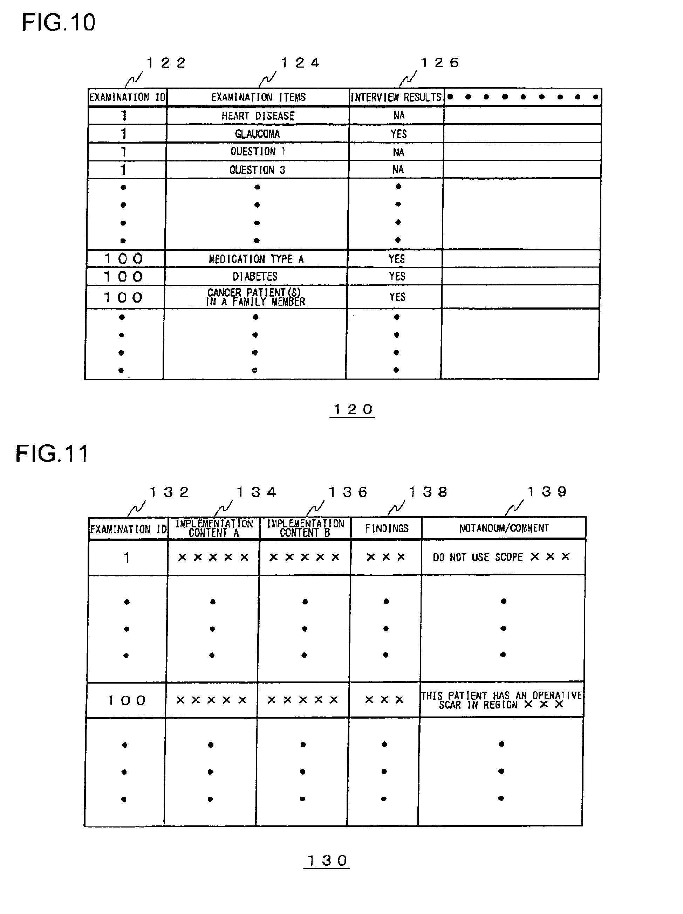

FIG. 10 shows an example of an interview sheet database 120, which is a database of interview sheet data. The interview sheet database 120 manages interview items 124, interview results 126, and the like, using examination IDs 122 as the key. When there is no answer to an interview item 124, "NA (not available)" is entered in the space of the interview results 126.

FIG. 11 shows an example of a report information database 130, which is a database of report information. The report information database 130 manages implementation contents A 134, implementation contents B 136, findings 138, and notandums and comments (notes and comments) 139, and the like for each examination, using examination IDs 122 as the key. The report information includes "examination ID" or "treatment ID", "reporting date", "reporter", "diagnosis", "findings", "implementation contents", "notandums and comments (notes and comments)", "post-examination notes and instructions" and so forth, as appropriate.

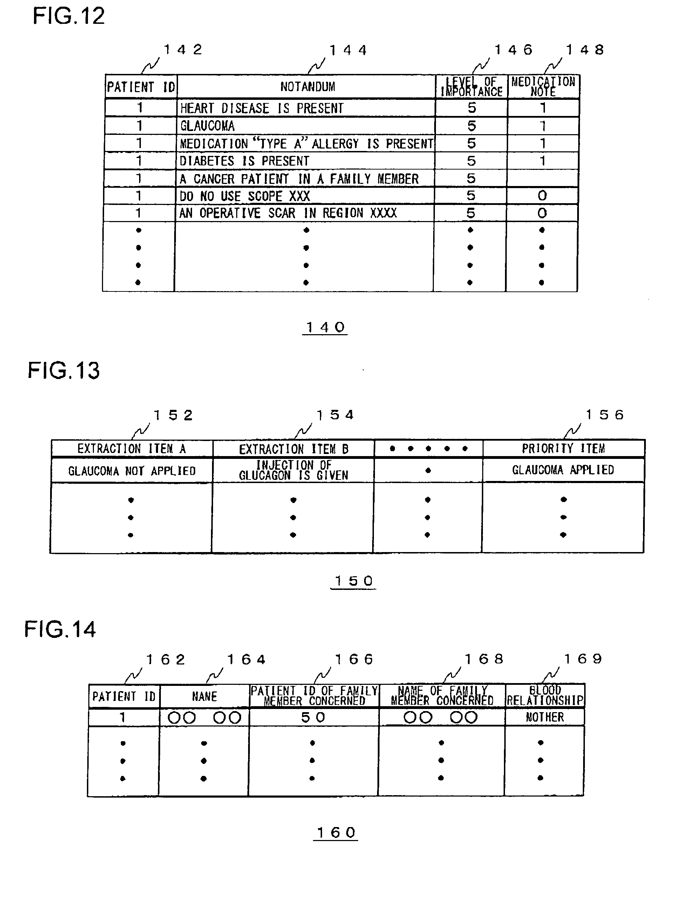

FIG. 12 shows an example of a patient-related information database 140, which is a database of patient-related information. The patient-related information database 140 manages notandums (notes) 144, importance levels 146, medication notes 148, and the like, using patient IDs 142 as the key. The patient-related information database 140 is prepared by extracting necessary contents from the patient information database 80, order information database 90, implementation information database 110, interview sheet database 120, and report information database 130. The details of this preparation method will be discussed later. The preparation unit 24 can prepare interview results by using the patient-related information database 140. The patient-related information is information concerning the subject's constitution, such as allergic predisposition, the subject's or the family's medical history, and the like, which is intended to provide the medical action performer with knowledge about forbidden medication or treatment and such other conditions for the patient.

The notandums or notes are displayed at each terminal when the medical action performers have received a patient and registered an examination order from the PC 40 to the medical information management apparatus 10 or when they registered the name of medication, such as a painkiller or an anesthetic, as a treatment before examination from the PDA 50. The importance level 146, which indicates the importance level of each applicable note, informs the medical action performers of the degree of necessity which is to be presented thereto. In FIG. 12, there are five levels of importance 146, "5" indicating the highest level of importance of noted information. For example, the importance "5" may be displayed in a large character or a red character at the terminal. Also, where the display of the PDA 50 is so small that all the notes cannot be displayed at the same time, the arrangement may be such that those notes with higher importance level only are displayed in the order of importance. The medication notes 148 show whether any specific note concerns medication or not. The medication notes are so important that the classification thereof must be readily recognizable by both the medical personnel and system.

The patient-related information generator 70 generates a patient-related information database 140 by extracting necessary contents from the patient information database 80, the order information database 90, the implementation information database 110, the interview sheet database 120, and the report information database 130. In such a process, there are cases where conflicting contents concerning the same matter are extracted from a plurality of databases. For example, while one database provides contents indicating the absence of glaucoma, the other database provides contents indicating the presence thereof in a patient. In such a case, priority is given to a safer side, so that medical action is taken in an inhibitory and controlled manner. In the above example, applicability of glaucoma is registered in the patient-related information database 140.

FIG. 13 shows a priority order table 150. The priority order table 150 predefines a priority order to be used when conflicting contents are extracted from a plurality of databases as mentioned above. The priority order table 150 manages a plurality of extraction items, such as extraction items A 152 and extraction items B 154 and priority items 156. FIG. 13 shows a case where an item indicating the nonapplicability of glaucoma has been extracted from the interview sheet database 120 and an item indicating a glucagon injection performed has been extracted from the implementation information database 110. The fact that glucagon has been injected indicates that the patient has glaucoma. In preparation for cases like this, where conflicting items are extracted, the safer side of the applicability of glaucoma is registered beforehand in the priority order table 150 as a priority item.

The relatives information is information concerning the constitution or medical history of a patient related by blood to a patient. When conducting a medical treatment or action on a patient, the physician takes the hereditary information or the like on him/her into consideration. Accordingly, the information on the patient's relatives accumulated in the interview sheet database 120 or the patient-related information database 140 will provide useful information.

FIG. 14 shows an example of a relatives information database 160, which is a database of relatives information. The relatives information database 160 manages names 164, patient IDs 166 of the family member concerned, the name 168 of the family member concerned, the blood relationship 169, and the like for each patient, using patient IDs 162 as the key. The relatives information database 160 may be prepared by collecting information not only from one's own department system but also from the other department systems.

When an interview result for an interview item concerning a relative is to be extracted from the relatives information database 160, the relatives information acquiring unit 72 extracts a patient ID 166 of the family member concerned using the patient ID 162 as the key. Then the relatives information acquiring unit 72 extracts implementation information on the patient from part or all of implementation information databases being managed in a medical institution, based on the extracted patient ID 166, and extracts medical history information concerning the relative from the implementation information as the interview result for the interview item. The preparation unit 24 prepares items concerning the relative in the interview sheet data, based on the interview result and the interview item corresponding thereto.

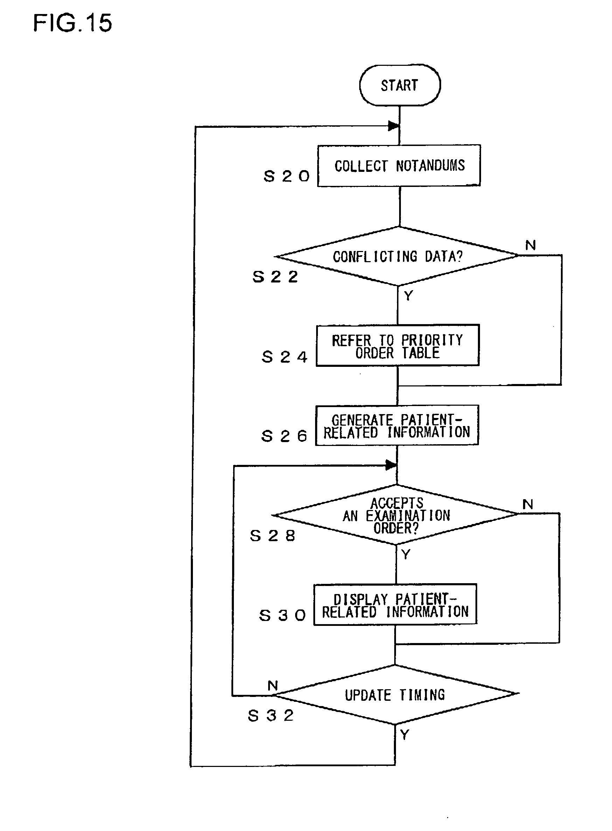

FIG. 15 is a flowchart to explain an operation of a medical information management apparatus 10 according to the second embodiment. The patient-related information generator 70 extract information to be presented to the medical action performers as important notes from the patient information database 80, the order information database 90, the implementation information database 110, the interview sheet database 120, and the report information database 130 (S20). For this information extraction, extraction items may be preset for each of the databases. Next, if there is a conflict among a plurality of extracted notes (Y of S22), the patient-related information generator 70 determines and selects notes with higher priority by referring to the priority order table 150 (S24). If there is no conflict among the notes (N of S22), the above processing is skipped. In this manner, the patient-related information generator 70 generates the patient-related information database 140 (S26).

When the medical action performer inputs a patient ID from the PC 40, after accepting a patient, in order to register an examination order to the medical information management apparatus 10 (Y of S28), the search unit 26 searches within the patient-related information database 140 for patient-related information on the applicable patient, using the patient ID as the key. Then the patient-related information is sent to the PC 40 and displayed on the display unit 42 (S30).

With the arrival of update timing of the patient-related information database 140 (Y of S32), the patient-related information generator 70 collects necessary notandums from the patient information database 80, the order information database 90, the implementation information database 110, the interview sheet database 120, and the report information database 130 (S20) and updates the contents of the patient-related information database 140. The patient-related information generator 70 carries out the updating at the periodic batch processing during the night or the registration of data in any of the databases.

According to the present embodiment as described above, notandums on each patient are collected into a database and managed in a unified manner, so that notes on forbidden medication for a patient and the like can be easily searched for and used in medical actions. Also, any risky medical actions can be prevented by displaying these notandums at the terminals used by the medical personnel. Also, since the system prepares notandums on patients by collecting information from a variety of databases, information more reliable than hand-written memoranda can be presented to the medical personnel. Moreover, the reliability of the contents is high because the database managing such notes in a unified manner is updated at regular intervals.

The present invention has been described based on the embodiments. These embodiments are merely exemplary, and it is understood by those skilled in the art that various modifications to the combination of each component and each process thereof are possible and that such modifications are also within the scope of the present invention.

The embodiment described above has been explained mainly on a system used in the endoscopic examination department. However, the system can be applied to other hospital department as well. In particular, the present embodiments can be used effectively in systems that involve a variety of examinations prior to the performance of treatment. The more the types of examination and the more the implementation information accumulated, the more will be the answers to a variety of interview items available from the implementation information database. For example, a variety of examinations are performed before surgery in the surgical department. Hence, the medical personnel in the department can greatly benefit from the reduced trouble of preparing interview sheet data. Furthermore, the presence of more reliable answers can raise the accuracy of the interview sheet database as a whole.

While the preferred embodiments of the present invention have been described using specific terms, such description is for illustrative purposes only, and it is to be understood that changes and variations may be made without departing from the spirit or scope of the appended claims.

## Claims

1. A medical information management apparatus (10), comprising:
a recording unit (30) which records implementation information on a medical action performed on a patient;
an extraction unit (22) which extracts information necessary to prepare an interview result of an interview item for the patient, from the implementation information recorded in the recording unit (30); and
a preparation unit (24) which prepares the interview result by referring to the information extracted by the extraction unit (22).

2. A medical information management apparatus (10) according to Claim 1, wherein the preparation unit (24) prepares interview sheet data from the interview result and the interview item corresponding thereto.

3. A medical information management apparatus (10) according to Claim 2, wherein the recording unit (30) records notandum information for the patient, and
wherein the extraction unit (22) extracts information necessary for preparing the interview result, by using the notandum information.

4. A medical information management apparatus (10), comprising:
a table (60, 66) which manages a medical action and an interview result for an interview item derived from the medical action by associating the medical action with the interview result;
a search unit (26) which conducts a search on whether a medical action performed on a patient corresponds to the medical action managed by the table (60, 66) or not; and
a preparation unit (24) which prepares interview sheet data from the interview result associated with the medical action and the interview item corresponding thereto when there exists the corresponding medical action as a result of the search by the search unit (26).

5. A medical information management apparatus according to any one of claims 2 to 4, further comprising a patient-related information generator (70) which generates patient-related information for each patient by extracting notandum information for a patient contained in at least one of the interview sheet data, the implementation information, report information and order information.

6. A medical information management apparatus according to Claim 5, wherein when conflicting notandum information is extracted from the interview sheet data, the implementation information, the report information and the order information, the patient-related information generator (70) gives priority to safer information for the patient among the conflicting notandum information.

7. A medical information management apparatus according to any one of claims 2 to 6, further comprising a relatives information acquiring unit (72) which acquires medical information concerning patient's relatives; and
a family medical history information generator (74) which generates family medical history information based on the acquired medical information concerning patient's relatives,
wherein the preparation unit (24) prepares the interview sheet data by referring to the interview result, the interview item corresponding thereto and the family medical history information.
